(19) Europäisches Patentamt
European Patent Office
Office européen des brevets

(11) Veröffentlichungsnummer: **0 050 229**
**B1**

(12) # EUROPÄISCHE PATENTSCHRIFT

(45) Veröffentlichungstag der Patentschrift: **28.09.83**

(51) Int. Cl.³: **C 07 C 85/06**, C 07 C 85/08

(21) Anmeldenummer: **81107488.9**

(22) Anmeldetag: **21.09.81**

(54) **Verfahren zur Herstellung von primären aromatischen Aminen aus cyclischen Alkoholen und/oder Ketonen.**

(30) Priorität: **16.10.80 DE 3039085**

(43) Veröffentlichungstag der Anmeldung: **28.04.82 Patentblatt 82/17**

(45) Bekanntmachung des Hinweises auf die Patenterteilung: **28.09.83 Patentblatt 83/39**

(84) Benannte Vertragsstaaten: **AT CH DE FR GB IT LI NL SE**

(56) Entgegenhaltungen:
**FR-A-1 313 520**
**US-A-3 442 950**
**US-A-3 553 268**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Hupfer, Leopold, Dr., Waltershoehe 3, D-6701 Friedelsheim (DE)**
Erfinder: **Hoffmann, Werner, Dr., Ringstrasse 11 C, D-6701 Neuhofen (DE)**
Erfinder: **Baumann, Manfred, Dr., Im Sennteich 26, D-6800 Mannheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 050 229 B1

BUNDESDRUCKEREI BERLIN

Verfahren zur Herstellung von primären aromatischen Aminen aus cyclischen Alkoholen und/oder Ketonen

Die älteste und zur Zeit immer noch wichtigste Methode zur Herstellung von primären aromatischen Aminen ist die Reduktion von aromatischen Nitroverbindungen (Houben-Weyl, »Methoden der organischen Chemie«, 4. Aufl. Band 11/1, S. 360 ff.). Nachteilig ist, daß die vorausgehende Nitrierung bei substituierten Aromaten wenig selektiv verläuft und daher oft Produktgemische auftreten.

Eine Möglichkeit, um zu einheitlichen primären aromatischen Aminen zu kommen, bietet die Umsetzung von Ammoniak mit cycloaliphatischen (alicyclischen) Verbindungen geeigneten Substitutionsmusters, beispielsweise von Derivaten des Cyclohexanols, Cyclohexenols, Cyclohexenons oder Cyclohexanons zu entsprechend substituierten Anilinen, wobei die Vorstellung besteht, daß die Hydroxygruppe zunächst durch Aminolyse in eine Aminogruppe (bzw. die Carbonylgruppe zunächst in eine Iminogruppe) überführt und unter Dehydrierung des Cyclohexanringes ein aromatischer Ring ausgebildet wird. In Houben-Weyl, a. a. O. S. 126 ff. bzw. S. 611 ff. und in Houben-Weyl Band 4/2, S. 338 ff. sind die entsprechenden Überlegungen angestellt (vgl. auch US-PS 3 553 268).

In den US-Patentschriften 3 219 702 und 3 219 704 ist die Synthese von aromatischen Aminen aus alicyclischen Ketonen und Ammoniak, primären oder sekundären Aminen in Gegenwart von Dehydrierungskatalysatoren beschrieben. Die dort angegebenen Verfahren haben aber den Nachteil, daß zum Teil Wasserstoffakzeptoren (US-PS 3 219 702) benötigt werden und daß viele Nebenprodukte, beispielsweise Cyclohexylamine, N-Cyclohexyl-aniline oder Dicyclohexylamine gebildet werden.

In der US-Patentschrift 3 553 268 wird die Herstellung von Anilin aus Gemischen von Cyclohexanol, Cyclohexanon und Ammoniak mit einem Nickelkatalysator beschrieben. Es soll dabei zwar eine hohe Ausbeute erreicht werden, jedoch scheint das Verfahren einmal auf substituierte Produkte nicht anwendbar, zum anderen wird es dadurch eingeschränkt, daß nur Gemische mit höchstens 65% Cyclohexanol verwendbar sind. Außerdem ist ein solches Verfahren gerade für die Herstellung von Anilin weniger interessant, da Anilin bekanntlich am billigsten aus Nitrobenzol erhältlich ist.

Die US-Patentschriften 3 442 950 und 3 347 921 beschäftigen sich ebenfalls mit der Umsetzung von Gemischen von Cyclohexanol und Cyclohexanon und Ammoniak oder primären Aminen an Dehydrierungskatalysatoren. Probleme entstehen bei diesen Verfahren durch höhersiedende Produkte und andere Nebenprodukte, u. a. Schiffsche Basen des Anilins und Cyclohexylamins, Phenol, Diphenylamin und Phenyl-cyclohexylamin, so daß eine aufwendige Verfahrensführung erforderlich wäre.

Aus der Tatsache, daß für die Herstellung von aromatischen Aminen z. B. schon die Umsetzung eines Gemisches von Phenolen und Cyclohexanonderivaten (wobei letztere als Katalysatoren dienen sollen) mit Ammoniak vorgeschlagen wurde (vgl. US-Patentschriften 3 960 962, 3 931 298 und DE-OS 2 208 827) ist zu ersehen, daß für einen industriell gangbaren Syntheseweg zu aromatischen Aminen ein Bedürfnis besteht.

Aufgabe der Erfindung ist die Schaffung eines Verfahrens mit dem man einheitliche, vorzugsweise substituierte aromatische Amine aus entsprechenden cyclischen Alkoholen und/oder Ketonen mit Hilfe von Ammoniak ggf. Wasserstoff und einem hydrierend/dehydrierend wirkenden Katalysator enthält. Eine besondere Aufgabe der Erfindung besteht darin, einen Katalysator vorzusehen, der sowohl Cyclohexanole als auch Cyclohexanone als auch deren Mischungen umzusetzen vermag.

Es wurde gefunden, daß diese Aufgabe befriedigend gelöst werden kann, wenn man einen Palladiumkatalysator verwendet, der auf einem Träger aufgebracht ist und außerdem Zink und/oder Cadmium enthält.

Nach der Erfindung erhält man z. B. Amine der allgemeinen Formel (I)

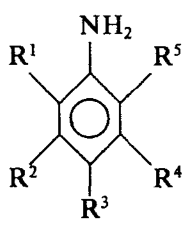

(I)

in der $R^1$ bis $R^5$ jeweils ein Wasserstoffatom oder gleiche oder verschiedene oder jeweils zu mehreren gemeinsam einen Substituenten bedeuten können. Aliphatische Gruppen mit 1 bis 20 Kohlenstoffatomen, cycloaliphatische Gruppen mit 5 bis 20 Kohlenstoffatomen, Arylgruppen mit 6 bis 20 Kohlenstoffatomen oder Aralkyl- oder Arylalkylgruppen mit 7 bis 20 Kohlenstoffatomen sind Beispiele für derartige Substituenten. Diese können gegebenenfalls Sauerstoff und/oder Stickstoff als Heteroatome enthalten oder es können zwei Reste einen Substituenten dadurch bilden, daß sie durch eine Molekülbrücke miteinander verbunden sind. Entsprechend können die Ausgangsprodukte

als cyclische Alkohole bzw. Ketone der allgemeinen Formel II, IIa

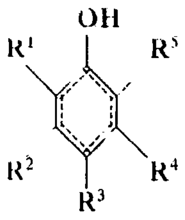

(II)

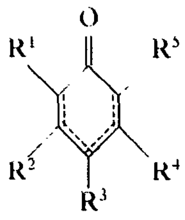

(II a)

in der $R^1$ bis $R^5$ jeweils entsprechende Bedeutung haben beschrieben werden. Das zentrale Ringgerüst kann gesättigt sein oder olefinisch ungesättigt; die Lage einer oder auch zweier Doppelbindungen kann beliebig sein, wie dies die gestrichelten Linien in (II) bzw. (IIa) angeben.

Überraschend ist, daß das erfindungsgemäße Verfahren mit Palladium allein nicht befriedigend betrieben werden kann, sondern man erst durch Zusätze Katalysatoren erhält, die so selektiv sind, daß die gewünschten aromatischen Amine in hoher Ausbeute und ausgezeichneter Reinheit anfallen; dies ist um so erstaunlicher, als man erwarten mußte, daß die Ausgangsstoffe (vgl. US PS 3 347 792) in vielfältiger Weise reagieren können: Die Dehydrierung zu den entsprechenden Phenolen, Umsetzung mit schon gebildeten aromatischen Aminen oder den als Zwischenstufe auftretenden Iminen zu Cyclohexylaminen, Bildung von Schiff'schen Basen und Hydrierung zu sekundären Aminen wären nur einige der möglichen Quellen für Nebenprodukte. Die Unterschiede, die verschieden zusammengesetzte Katalysatoren auf den Reaktionsablauf ausüben, können anhand von Vergleichsversuchen mit dem oft empfohlenen Raney Nickel und normalen Palladium Katalysatoren leicht gezeigt werden; ein typisches Beispiel für den Ablauf an einem gewöhnlichen Palladium Katalysator gibt der Vergleichsversuch zu Beispiel 14 wieder.

Es besteht die Vorstellung, daß bei dem erfindungsgemäßen Verfahren die Anlagerung des Ammoniaks relativ rasch und weitgehend vollständig abläuft, bevor die energetisch gleichermaßen begünstigte direkte Dehydrierung des Cyclohexanols bzw. Ketons einsetzt, so daß die Reaktion zum aromatischen Amin und nicht zum Phenol verläuft.

Offenbar findet mit den erfindungsgemäßen Katalysatoren unter den Reaktionsbedingungen auch keine Hydrierung der Zielprodukte oder Weiterreaktion der als Zwischenstufe auftretenden Cyclohexylamine zu bicyclischen Verbindungen statt.

Die als Ausgangsstoffe benötigten cyclischen Alkohole kann man auf einfache Weise durch Hydrierung von Phenolen oder Cyclohexanonen gewinnen. Die Ketone können z. B. durch Umsetzung von anderen Ketonen mit $\alpha,\beta$ ungesättigten Aldehyden oder gleichen oder verschiedenen Ketonen erhalten werden (Houben Weyl), a. a. O. Band 7/2b, S. 1629 1634, Band 8, Seiten 595 - 597, Org. Reactions 10, (1959), S. 179 555). Gesättigte cyclische Ketone können durch Partialhydrierung ungesättigter Ketone hergestellt werden (s. Beispiel 12). Schließlich können technische Gemische von Cyclohexanolen und Cyclohexanonen verwendet werden und zwar von beliebiger Zusammensetzung, wie sie z. B. durch Oxidation von Cyclohexanen mit Sauerstoff anfallen.

Für das erfindungsgemäße Verfahren eignen sich beispielsweise folgende cyclische Ketone:

Cyclohexanon, 2 Methylcyclohexanon, 3 Methylcyclohexanon, 4 Methylcyclohexanon, Cyclohex 2 en 1 on, 2-Methyl cyclohex 2 en-1 on, 3 Methyl cyclohex 2-en 1 on, 4 Methyl cyclohex 2-en 1-on, 2,6-Dimethylcyclohexanon, 2,6 Dimethylcyclohex-2-en-1-on, 3,5 Dimethyl cyclohexanon, 3,4 Dimethylcyclohexanon, 2,3,6-Trimethyl cyclohex-2-en-1-on, 2,6 Diisopropylcyclohexanon, 2 Methyl-6 ethyl cyclohexanon, 2-Methyl-6 ethyl-cyclohex-2-en-1 on, 2 Methyl 6 butyl cyclohexanon, 2-Dodecylcyclohexanon, 2-Methyl-6-cyclohexyl cyclohexanon, 2,6 Dimethyl 3-phenyl-cyclohex-5 en-1-on, 2,6 Dimethyl 3-(p methylphenyl)-cyclohex-5-en 1 on, 2,6 Dimethyl 3 (p methoxyphenyl)-cyclohex-5-en 1-on, 2 Methyl tetralon, (2 Methyl-2 H-3,4 dihydro-naphthalin 1 on), Tetralon.

Als cyclische Alkohole kommen z. B. in Betracht:

Cyclohexanol, 2-Methylcyclohexanol, 3-Methylcyclohexanol, 4-Methylcyclohexanol, 2,6-Dimethylcyclohexanol, 3,5-Dimethylcyclohexanol, 3,4-Dimethylcyclohexanol, 2,3-Dimethylcyclohexanol, 2,3,6-Trimethylcyclohexanol, 2,4,6-Trimethylcyclohexanol, 2,6-Diethylcyclohexanol, 2,6-Diisopropylcyclohexanol, 2,6-Di-sec.-butyl-cyclohexanol, 2-tert.-Butylcyclohexanol, 2-Methyl-6-ethyl-cyclohexanol, 2-Methyl-6-isopropyl-cyclohexanol, 2-Methyl-6-sec.-butyl-cyclohexanol, 2-Dodecylcyclohexanol, 2-Methyl-6-cyclohexanol, 2,6-Dimethyl-3-phenyl-cyclohexanol, 2,6-Dimethyl-3-(p-methylphenyl)-cyclohexanol, 2,6-Dimethyl-3-(p-methoxyphenyl)-cyclohexanol, 2-Methyl-1,2,3,4-tetrahydro-naphth-1-ol.

Der erforderliche Ammoniak kann — bezogen auf das Keton — in stöchiometrischer Menge oder in einem beliebigen Überschuß angewendet werden. Bei kontinuierlicher Arbeitsweise hat es sich als vorteilhaft erwiesen, gemeinsam mit Ammoniak Wasserstoff über den Katalysator zu leiten. Das Verhältnis von Ammoniak zu Wasserstoff liegt z. B. zwischen 1000 : 1 bis 1 : 10, d. h. stöchiometrische Mengen an Wasserstoff sind vom Ergebnis des Einzelexperiments her nicht notwendig. Es hat sich allerdings gezeigt, daß bei lange Zeit fortgesetzten Versuchen der Katalysator eine längere Lebensdauer hat, wenn man einen gewissen Überschuß an Wasserstoff, z. B. bis zum 10fachen der molaren Menge an Ammoniak anwendet. Verwendet man z. B. in den Versuchen der nachstehenden Beispiele das Verhältnis von Wasserstoff zu Ammoniak in diesem Sinne, so ist das Ergebnis nach Umsatz und Ausbeute bzw. Selektivität im wesentlichen unverändert (insbesondere werden keine größeren Mengen an Hydrierungsprodukten gebildet), jedoch erhöht sich die Katalysatorstandzeit im Dauerversuch deutlich.

Die Umsetzung ist sowohl kontinuierlich als auch diskontinuierlich in der Gas- oder Flüssigphase durchführbar. Bevorzugt wird für die Umsetzung genügend leicht verdampfbarer Ausgangsstoffe bzw. -gemische eine kontinuierliche Betriebsweise in der Gasphase. Es kann bei atmosphärischem Druck oder bei einem Druck bis zu 300 bar gearbeitet werden. Als Reaktionstemperatur sind 100 bis 350°C, insbesondere 150 bis 300°C geeignet. Unterhalb von 200°C wird in manchen Fällen — besonders bei Cyclohexanolen — ein unvollständiger Umsatz beobachtet, da die Dehydrierung der Amine höhere Temperatur bevorzugt.

Wenn man ausgeht von olefinisch ungesättigten Verbindungen, so kann die Umsetzung am gleichen Katalysator auch zweistufig vorgenommen werden, indem man zunächst in Abwesenheit von Ammoniak lediglich hydriert (vergleiche Beispiel 12) und das hydrierte Produkt dann in der erfindungsgemäßen Weise umsetzt.

Die Umsetzung in flüssiger Phase kann ohne Lösungsmittel oder in Anwesenheit von Lösungsmitteln, die unter den Reaktionsbedingungen inert sind, durchgeführt werden. Als Lösungsmittel kommen beispielsweise in Frage: Methanol, Ethanol, n-Butanol, Tetrahydrofuran, Dioxan, Anisol, Cyclohexylmethylether, 1,2-Dimethoxyethan, Toluol, Xylol, Cyclohexan. Man kann auch einen Überschuß an gebildetem Amin verwenden, d. h. einen Kreislauf des Reaktionsgemisches vorsehen; in diesem Fall ist ein Überschuß an Ammoniak empfehlenswert.

Beim Umsetzen von olefinisch ungesättigten Ketonen (Derivaten des Cyclohexenons) nach dem erfindungsgemäßen Verfahren ist es ratsam, die Reaktion in Anwesenheit einer Base, z. B. eines tertiären Amins oder vorzugsweise in einem tert. Amin als Lösungsmittel vorzunehmen, da dadurch die Dehydrierung der Ketone zu entsprechenden Phenolen unterdrückt wird (Beispiel 4, 7). Geeignete tertiäre Amine sind etwa Triethylamin, Tri-n-propylamin, Tri-n-butylamin, N,N-Dimethyl-cyclohexylamin, N-Methylpiperidin, N-Ethylpiperidin, N-Methylmorpholin, N-Methylpyrrolidin. Diese Stoffe werden entweder in geringerer Menge oder aber, als Lösungsmittel, in einem Gewichtsverhältnis von bis zu 10 : 1 bezogen auf das Keton eingesetzt.

Das nach dem Verfahren der Erfindung verwendete Katalysatorsystem enthält einerseits Palladium und andererseits Zink oder Cadmium allein oder — bevorzugt — im Gemisch miteinander. Die Bestandteile des Katalysators sind auf einem Träger aufgebracht, der aus Aluminiumoxid, Kieselsäure, Aluminiumsilikat, Magnesiumsilikat, Spinellen des Aluminiums, Chroms oder Eisens oder Aktivkohle bestehen kann. Bevorzugt ist die Verwendung von Aluminiumoxid. Die Zusätze, die dem Katalysator seine Eigenschaften verleihen, können sich an der Oberfläche des Katalysators befinden oder mit dem ursprünglichen Träger durch nachträgliches Tempern ein gemeinsames Kristallgitter bilden (beispielsweise bei Aluminiumoxidträgern: Bildung von typischen Spinellgittern mit Zink). Das bedeutet, daß die Träger Aktivität und auch Lebensdauer des Katalysators günstig beeinflussen.

Die Zusätze können gemeinsam mit Palladium durch Tränken des Trägers mit Lösungen ihrer z. B. Nitrate, Chloride, Formiate oder Oxalate aufgebracht werden. Durch anschließende thermische Behandlung, die gewöhnlich zwischen 400 und 600°C durchgeführt wird, erhält man ein Oxidgemisch. Soll bei Aluminiumoxidträgern, beispielswise mit Zink, Spinellbildung erzielt werden, muß nach der Tränkung auf eine Temperatur von 900 bis 1300°C erhitzt werden (siehe Ullmanns Enzyklopädie der technischen Chemie, 3. Auflage [1955] Band 6, S. 242—244, Gmelin, System-Nr. 35, Al, Tl 1934—1935, S.

26—28) und anschließend in der üblichen Weise das Palladium aufgebracht werden. Es ist daher auch ohne Belang, welchen Wertigkeitszustand Zink und Cadmium auf dem Katalysator einnehmen.

Der Palladiumgehalt des Katalysators ist nicht kritisch und kann in weiten Grenzen variiert werden. Zweckmäßig ist ein Gehalt von 0,05 bis 15 Gew.-%, bezogen auf das Trägermaterial; Zink und Cadmium werden z. B. in einer Menge von insgesamt 0,01 bis 5 Gew.-%, bezogen auf den Träger angewendet. Das Gewichtsverhältnis von Zink und Cadmium zu Palladium kann somit beispielsweise zwischen 10 000 : 1 bis 1 : 1500, bevorzugt 100 : 1 bis 1 : 50 liegen. Man verwendet den Katalysator wie üblich, beispielsweise in Form von Strängen die z. B. etwa 3 bis 5 mm Durchmesser und 10 mm Länge haben können oder als Pulver. Die angewendete Form richtet sich in üblicher Weise nach der vorgesehenen Verfahrenstechnik.

Praktisch verfährt man z. B. wie folgt: Auf strang- oder pulverförmiges $\gamma$-Aluminiumoxid wird Palladium zusammen mit dem Zusatzelement (Zink, Cadmium) in der gewünschten Menge durch Tränken mit beispielsweise deren wäßrigen Nitrat- oder Formiatlösungen aufgebracht und bei 150° C getrocknet. Danach wird 6 Stunden bei 550° C getempert und im Wasserstoffstrom bei 180° C reduziert. — Soll ein Katalysator mit der Struktur eines Spinells hergestellt werden, so wird zunächst nur Zink und bzw. oder Cadmium auf $\alpha$-Aluminiumoxid durch Tränken aufgebracht und dann 6 Stunden lang bei 1050° C geglüht. Dieser Träger wird dann mit wäßriger Palladiumnitratlösung getränkt und durch Erhitzen bei 180° C im Wasserstoffstrom reduziert. Wird zum Tränken Palladium-(II)-chloridlösung genommen, kann mit alkalischer Formalinlösung oder 5%iger wäßriger Hydrazinlösung reduziert werden.

Palladiumkatalysatoren, die Zink oder Cadmium oder beides enthalten, sind im übrigen auch bekannt; sie wurden aber bisher für andere Zwecke, z. B. für spezielle Hydrierungen verwendet. Angegeben werden solche Katalysatoren z. B. in der DE-PS 1 115 238 der DE-OS 2 139 574 und der DE-AS 2 431 929.

Die nach dem Verfahren der Erfindung hergestellten Verbindungen werden z. B. als Zwischenprodukte für Wirkstoffe in Pflanzenschutzmitteln benötigt (DE-AS 2 305 595, DE-OS 2 648 008, DE-OS 2 513 732, DE-OS 2 515 091).

Die in den folgenden Beispielen genannten Teile bedeuten Gewichtsteile; Raumteile verhalten sich zu ihnen wie Liter zu Kilogramm. Siedepunktsangaben (Kp) ohne Index beziehen sich auf atmosphärischen Druck, der Index bedeutet mbar.

### Beispiel 1

In einem Rohrreaktor mit einem Fassungsvermögen von einem Liter, entsprechend 1000 Raumteilen wird ein strangförmiger Katalysator angeordnet, der 0,5 Gew.-% Pd, 0,11 Gew.-% Zn und 0,1 Gew.-% Cd auf $Al_2O_3$ als Träger enthält, und auf 210° C gebracht. Über dieses Katalysatorbett werden stündlich bei atmosphärischem Druck eine Gasmischung aus 400 000 Raumteilen Ammoniak und 10 000 Raumteilen Wasserstoff und im Gleichstrom 100 Teile 2,6-Dimethylcyclohexan-1-on (gasförmig) geleitet. Das Reaktionsprodukt wird soweit gekühlt, daß die hochsiedenden Bestandteile flüssig werden und destilliert werden können, die gasförmigen Bestandteile werden im Kreis geführt. Man erhält aus 100 Teilen 2,6-Dimethylcyclohexan-1-on 92 Teile 2,6-Dimethylanilin (Xylidin) (Kp = 216° C), entsprechend einer Ausbeute von 96% der berechneten Ausbeute.

### Beispiel 2

Wie in Beispiel 1 beschrieben, werden 100 Teile 2,3,6-Trimethylcyclohexan-1-on pro Stunde — bei 230° C, unter sonst gleichen Bedingungen — umgesetzt. Man erhält hierbei 2,3,6-Trimethylanilin, $Kp_{26}$ = 119° C in einer Ausbeute von 93%.

### Beispiel 3

Man verfährt analog Beispiel 1, verwendet aber als Ausgangsstoff Tetralon. Es wird $\alpha$-Naphthylamin (Fp. 49° C) in einer Ausbeute von 95% erhalten.

### Beispiel 4

In einem Wirbelbettreaktor mit einem Fassungsvermögen von 1,3 Litern entsprechend 1300 Raumteilen werden 1000 Raumteile Katalysator eingefüllt. Der Katalysator besteht aus 0,5 Gew.-% Pd, 0,11 Gew.-% Zn, 0,1 Gew.-% Cd auf $Al_2O_3$ und besitzt eine Korngröße von 0,2 bis 0,6 mm. Die Temperatur des Reaktors wird auf 230° C eingestellt und eine entsprechend vorerhitzte Mischung aus 200 000 Raumteilen Ammoniak und 10 000 Raumteilen Wasserstoff pro Stunde eingeleitet. In die so

erzeugte Katalysator-Wirbelschicht wird stündlich und kontinuierlich eine Lösung von 50 Teilen 2,3,6-Trimethylcyclohex-5-en-1-on in 50 Teilen N-Methylpiperidin eingesprüht. Das Reaktionsprodukt wird durch Kühlung der Abgase gewonnen und destilliert. Aus je 100 Teilen 2,3,6-Trimethyl-cyclohex-5-en-1-on werden 89 Teile 2,3,6-Trimethylanilin erhalten, Ausbeute: 91%.

Beispiel 5

Man verfährt analog Beispiel 4, verwendet aber als Ausgangsstoff 3-Methyl-cyclohex-2-en-1-on. Als Endprodukt wird m-Toluidin (Kp = 203° C) in einer Ausbeute von 93% erhalten.

Beispiel 6

Man verfährt analog Beispiel 4, setzt aber Cyclohex-2-en-1-on als Ketonkomponente zur Reaktion ein. Das führt zu Anilin (Kp = 184° C) als Umsetzungsprodukt, in einer Ausbeute von 88%.

Beispiel 7

In einem Rührautoklaven mit einem Nutzvolumen von 300 Raumteilen wird eine Lösung von 35 Teilen 2,3,6-Trimethylcyclohex-5-en-1-on, in 35 Teilen N-Methylmorpholin, 34 Teile Ammoniak und 5 Teile eines feinteiligen Katalysators, der 0,5 Gew.-% Pd und 0,1 Gew.-% Zn auf $Al_2O_3$ enthält, 10 Stunden lang auf 230° C und dem sich dabei einstellenden Eigendruck (ca. 150 bar) gehalten. Anschließend läßt man abkühlen, filtriert ab und reinigt durch Destillation. Man erhält hierbei 18,2 Teile 2,3,6-Trimethylanilin und 15,3 Teile 2,3,6-Trimethylcyclohexan-1-on, das vermutlich durch Partialhydrierung des eingesetzten 2,3,6-Trimethylcyclo-hex-5-en-1-ons mit dem während der Reaktion entstandenen Wasserstoff gebildet wird. Da 2,3,6-Trimethylcyclohexan-1-on wiederum in das gewünschte 2,3,6-Trimethylanilin überführt werden kann, beispielsweise nach Beispiel 1 oder 2, beträgt die Selektivität der Reaktion 96%.

a) Vergleichsversuch zu Beispiel 7

Entsprechend der Ausgestaltung des Beispiels 7 wird in einem Rührautoklaven mit 300 Raumteilen Nutzraum eine Lösung von 35 Teilen 2,3,6-Trimethylcyclohex-5-en-1-on, in 35 Teilen N-Methylmorpholin, 34 Teile Ammoniak und 5 Teile Raney-Nickel 10 Stunden lang bei dem sich einstellenden Eigendruck (ca. 150 bar) auf 230° C erhitzt. Man kühlt ab, filtriert und destilliert das Lösungsmittel ab. Das Reaktionsprodukt hat nach gaschromatographischer Analyse folgende Zusammensetzung: 11 Gew.-% 2,3,6-Trimethylanilin, 89 Gew.-% eines destillativ nicht trennbaren Gemisches aus Ausgangsprodukt, 2,3,6-Trimethylcyclohexan-1-on, 2,3,6-Trimethylcyclohexanol und 2,3,6-Trimethylcyclohexylamin.

b) Vergleichsversuch zu Beispiel 7

Man verfährt wie vorstehend beschrieben, verwendet aber anstatt Raney-Nickel einen Katalysator mit 10 Gew.-% Palladium auf Aktivkohle. Dieser Versuch liefert ein Reaktionsprodukt, das nach gaschromatographischer Analyse aus 38 Gew.-% Trimethylanilin und 62 Gew.-% eines destillativ nicht trennbaren Gemisches aus Ausgangsprodukt, 2,3,6-Trimethylcyclohexan-1-on, 2,3,6-Trimethylcyclohexanol und 2,3,6-Trimethylcyclohexylamin besteht.

Beispiel 8

Man verfährt entsprechend Beispiel 7, verwendet aber als Ausgangsstoff 2,6-Dimethyl-3-phenyl-cyclohex-5-en-1-on. Es wird 2,6-Dimethyl-3-phenylanilin ($Kp_{0,2}$ = 121° C) mit einer Selektivität von 95% erhalten.

Beispiel 9

Man verfährt entsprechend Beispiel 7, verwendet aber als Ausgangsstoff 2,6-Dimethyl-3-(p-methylphenyl)-cyclohex-5-en-1-on. Es wird 2,6-Dimethyl-3-(p-methylphenyl)-anilin ($Kp_{0,3}$ = 132° C) mit einer Selektivität von 87% erhalten.

Beispiel 10

Man verfährt entsprechend Beispiel 7, verwendet aber als Ausgangsstoff 2,6-Dimethyl-3-(p-methoxyphenyl)-cyclohex-5-en-1-on. Es wird 2,6-Dimethyl-3-(p-methoxyphenyl)-anilin ($Kp_{0,4}$ = 151° C) mit einer Selektivität von 87% erhalten.

Beispiel 11

Man verfährt entsprechend Beispiel 7, verwendet aber als Ausgangsstoff 2-Ethyl-cyclohex-5-en-1-on. Es wird 2-Ethylanilin (Kp = 209° C) in einer Selektivität von 93% erhalten.

Beispiel 12

(Beispiel für eine Partialhydrierung eines olefinisch ungesättigten Ketons)

In einem Rührautoklaven mit einem Nutzraum von 5 Litern entsprechend 5000 Raumteilen wird eine Mischung aus 2500 Teilen 2,3,6-Trimethylcyclohex-5-en-1-on und 250 Teilen Katalysator der 0,5 Gew.-% Pd und 0,1 Gew.-% Zn auf $Al_2O_3$ enthält, zunächst bei 70, dann bei 100° C und jeweils 20 bar Wasserstoffdruck hydriert, bis der Druck konstant bleibt. Das flüssige Reaktionsgemisch wird filtriert, wobei 2440 Teile Filtrat erhalten werden, das zu 96% aus 2,3,6-Trimethylcyclohexan-1-on bestehen und ohne weiteres zur Herstellung von 2,3,6-Trimethylanilin (beispielsweise nach Beispiel 1) eingesetzt werden kann.

Beispiel 13

In ein druckfestes zylindrisches Rohr mit einem Volumen von 1,2 l als Reaktor wird ein Katalysator in Form von Strängen (3 mm Durchmesser, 10 mm Länge), der 1,8 Gew.-% Palladium auf einem Zink-Aluminium-Spinell als Träger enthält, eingefüllt und auf 120° C erhitzt, über dieses Katalysatorbett werden stündlich bei Atmosphärendruck eine Gasmischung aus 200 NL Ammoniak und 200 NL Wasserstoff und 100 g verdampftem 2,6-Dimethylcyclohexan-1-on geleitet. Das Reaktionsprodukt wird — sobald es den Reaktor verläßt — abgekühlt und destilliert. Man erhält aus 100 g 2,6-Dimethylcyclohexan-1-on 93 g 2,6-Dimethylanilin (Kp = 216° C), entsprechend einer Ausbeute von 97% d. Th.

Beispiel 14

In einem Rohrreaktor mit einem Fassungsvermögen von einem Liter, entsprechend 1000 Raumteilen, wird ein strangförmiger Katalysator (3 mm Durchmesser, 10 mm Länge) angeordnet, der 0,5 Gew.-% Palladium, 0,2 Gew.-% Zink und 0,1 Gew.-% Cadmium auf Aluminiumoxid als Träger enthält, und auf 220° C erhitzt. Über dieses Katalysatorbett werden stündlich bei Atmosphärendruck eine Gasmischung bestehend aus 200 000 Raumteilen Ammoniak und 200 000 Raumteilen Wasserstoff und im Gleichstrom 50 Teile 2,6-Dimethylcyclohexanol (gasförmig) geleitet. Das Reaktionsprodukt wird — sobald es den Reaktor verläßt — abgekühlt. Es besteht nach gaschromatographischer Analyse aus 1,3 Gew.-% m-Xylol, 2,6 Gew.-% 2,6-Dimethylcyclohexylamin und 96,1 Gew.-% 2,6-Dimethylanilin. Die Reinigung des 2,6-Dimethylanilins geschieht durch eine einfache Destillation (Kp = 216° C). Man erhält aus 100 Teilen 2,6-Dimethylcyclohexanol 89,5 Teile 2,6-Dimethylanilin, entsprechend einer Ausbeute von 94,5%.

Beispiel 15

Wie in Beispiel 14 beschrieben, werden 50 Teile 2,3,6-Trimethylcyclohexanol pro Stunde bei 240° C, unter sonst gleichen Bedingungen, umgesetzt. Man erhält hierbei 2,3,6-Trimethylanilin $Kp_{26}$ = 119° C, in einer Ausbeute von 92%.

Beispiel 16

Man verfährt entsprechend Beispiel 14, verwendet aber als Ausgangsstoff $\alpha$-Tetralol (1,2,3,4-Tetrahydro-1-naphthol). Es wird $\alpha$-Naphthylamin (Fp. 49° C) in einer Ausbeute von 94% gebildet.

Beispiel 17

Man verfährt entsprechend Beispiel 14, verwendet aber einen Katalysator, der 0,5 Gew.-% Palladium auf einem Zink-Aluminiumspinell enthält. Ausgehend von 2,6-Dimethylcyclohexanol wird unter diesen Bedingungen 2,6-Dimethylanilin in einer Ausbeute von 96% gebildet.

Beispiel 18

In einem Wirbelbettreaktor mit einem Fassungsvermögen von 1,2 Liter, entsprechend 1200 Raumteilen, werden 1000 Raumteile Katalysator eingefüllt. Der Katalysator besteht aus 0,5 Gew.-% Palladium, 0,2 Gew.-% Zink auf $Al_2O_3$ und besitzt eine Korngröße von 0,2 bis 0,6 mm. Die Temperatur des Reaktors wird auf 220°C eingestellt und eine entsprechend vorerhitzte Mischung aus 200 000 Raumteilen Ammoniak und 200 000 Raumteilen Wasserstoff pro Stunde eingeleitet. Durch die so erzeugte Katalysator-Wirbelschicht wird stündlich und kontinuierlich eine Mischung von 50 Teilen geführt, die aus 80 Gew.-% 2,6-Dimethylcyclohexanol und 20 Gew.-% 2,6-Dimethylcyclohexanon bestehen. Das Reaktionsprodukt wird durch Kühlung der Abgase gewonnen und destilliert. Aus je 100 Teilen der eingesetzten 2,6-Dimethylcyclohexanol/-on-Mischung werden 92 Teile 2,6-Dimethylanilin gewonnen, Ausbeute: 96,5%.

Beispiel 19

Man verfährt entsprechend Beispiel 18, verwendet aber als Ausgangsprodukt eine Mischung aus 60 Gew.-% Cyclohexanol und 40 Gew.-% Cyclohexanon. Das führt in einer Ausbeute von 93% zu Anilin (Kp = 184°C) als Umsetzungsprodukt.

Beispiel 20

Man verfährt entsprechend Beispiel 18, verwendet aber als Ausgangsprodukt 3-tert.-Butyl-cyclohexanol. Als Endprodukt wird m-tert.-Butylanilin ($Kp_{0,3}$ = 72 bis 73°C) in einer Ausbeute von 94% erhalten.

Beispiel 21

In einem Rührautoklaven mit einem Volumen von 300 Raumteilen wird eine Mischung aus 51 Teilen 2-Ethyl-cyclohexanol, 41 Teilen Ammoniak und 5 Teilen eines feinpulverigen Katalysators, der 10 Gew.-% Palladium, 0,2 Gew.-% Zink auf $Al_2O_3$ enthält, 10 Stunden lang auf 275°C erhitzt. Es stellt sich hierbei ein Eigendruck von ca. 200 bar ein. Anschließend läßt man abkühlen, filtriert ab und reinigt durch Destillation. Man erhält hierbei 22 Teile 2-Ethylanilin ($Kp_{760}$ = 209°C) und 25 Teile eines Gemisches aus 2-Ethylcyclohexanol und 2-Ethylcyclohexylamin. Da das 2-Ethyl-cyclohexanol/-cyclohexylamin-Gemisch, beispielswise beim Arbeiten analog den Beispielen 1 oder 14, ebenfalls in 2-Ethylanilin überführt werden kann, beträgt die Selektivität der Reaktion 95%.

Beispiel 22

Man verfährt entsprechend Beispiel 21, verwendet aber als Ausgangsstoff 2,6-Dimethyl-3-(p-methylphenyl)-cyclohexanol. Es wird 2,6-Dimethyl-3-(p-methylphenyl)-anilin ($Kp_{0,3}$ = 132°C) mit einer Selektivität von 91% erhalten.

Beispiel 23

Man verfährt entsprechend Beispiel 21, verwendet aber als Ausgangsstoff 2,6-Dimethyl-3-(p-methoxyphenyl)-cyclohexanol. Es wird 2,6-Dimethyl-3-(p-methoxyphenyl)-anilin ($Kp_{0,4}$ = 151°C) mit einer Selektivität von 85% erhalten.

Vergleichsversuch

Es wird entsprechend Beispiel 14 gearbeitet, aber ein Katalysator verwendet, der 0,5 Gew.-% Palladium auf Aluminiumoxid und keine Zusatzstoffe enthält. Das mit diesem Katalysator gewonnene Reaktionsprodukt hat nach gaschromatischer Analyse folgende Zusammensetzung: 5 Gew.-%

m-Xylol, 11 Gew.-% 2,6-Dimethylcyclohexanol, 8 Gew.-% 2,6-Dimethylphenol, 76 Gew.-% 2,6-Dimethylanilin.

**Patentansprüche**

1. Verfahren zur Herstellung von primären aromatischen Aminen der allgemeinen Formel $ArNH_2$, wobei Ar einen ggf. substituierten Arylrest darstellt, durch Umsetzung von entsprechenden gesättigten oder olefinisch ungesättigten cycloaliphatischen Alkoholen und/oder Ketonen, in denen die Hydroxyl- bzw. Oxogruppe einen Substituenten eines vorzugsweise 6gliedrigen, aromatisierbaren Rings bildet, mit Ammoniak in Gegenwart eines hydrierend/dehydrierenden Katalysators und vorzugsweise in Gegenwart von Wasserstoff, dadurch gekennzeichnet, daß man als Katalysator Palladium auf einem Träger verwendet, der außerdem Zink und/oder Cadmium enthält.

2. Verfahren nach Anspruch 1, dadurch gekennzeichnet, daß man, falls ein olefinisch ungesättigtes Keton vorliegt, in Anwesenheit einer Base umsetzt.

**Claims**

1. A process for the production of an aromatic amine of the general formula $ArNH_2$, Ar denoting optionally substituted aryl, by reacting a corresponding saturated or olefinically unsaturated cycloaliphatic alcohol and/or ketone in which the hydroxyl or oxo group forms a substituent of a preferably six-membered, aromatizable ring, with ammonia in the presence of a (de)hydrogenating catalyst and preferably in the presence of hydrogen, wherein palladium supported on a carrier and also containing zinc and/or cadmium is used as catalyst.

2. A process as claimed in claim 1, wherein, if an olefinically olefinically unsaturated ketone is employed, the reaction is carried out in the absence of a base.

**Revendications**

1. Procédé pour la préparation d'amines primaires aromatiques de formule générale $ArNH_2$, Ar représentant un radical aryle éventuellement substitué, par réaction d'alcools et/ou cétones cycloaliphatiques saturés ou oléfiniquement insaturés correspondants, dans lesquels le groupe hydroxyle ou céto constitue un substituant d'un noyau aromatisable, de préférence hexagonal, avec l'ammoniac, en présence d'un catalyseur d'hydrogénation/déshydrogénation et, de préférence, en présence d'hydrogène, caractérisé en ce qu'on utilise, en tant que catalyseur, du palladium sur un support, ce catalyseur contenant en outre du zinc et/ou du cadmium.

2. Procédé selon la revendication 1, caractérisé en ce que dans le cas d'une cétone insaturée oléfiniquement, on effectue la réaction en présence d'une base.